# EUROPÄISCHE PATENTSCHRIFT

(11) **EP 0 970 048 B1**
(45) Veröffentlichungstag und Bekanntmachung des Hinweises auf die Patenterteilung: **18.12.2002**
(21) Anmeldenummer: 98920470.6
(22) Anmeldetag: 04.03.1998
(51) Int. Cl.: C07C 409/38, C08F 4/34

(54) **T-BUTYLPEROXY-CYCLODODECYL-OXALAT**
T-BUTYLPEROXY-CYCLODODECYL-OXALATE
T-BUTYLPEROXY-CYCLODODECYL-OXALATE

(30) Priorität: 05.03.1997 DE 19708982
(43) Veröffentlichungstag der Anmeldung: 12.01.2000
(73) Patentinhaber: Peroxid-Chemie GmbH & Co. KG., 82049 Pullach (DE)
(72) Erfinder: HÄGEL, Eberhard, D-82057 Icking (DE); ZEISS, Werner, D-82547 Eurasburg (DE); DORN, Maximilian, D-82049 Pullach (DE)
(74) Vertreter: Huber, Bernhard, Dipl.-Chem.
(86) Internationale Anmeldenummer: EP9801215
(87) Internationale Veröffentlichungsnummer: WO98039294

(56) Entgegenhaltungen:
- EP-A- 0 095 860
- EP-A- 0 271 462

## Beschreibung

Die vorliegende Erfindung betrifft die neue Verbindung t-Butylperoxy-cyclododecyl-oxalat sowie ihre Herstellung und Verwendung als Polymerisationsinitiator.

Für die Polymerisation von ungesättigten Monomeren, insbesondere von Vinylchlorid, besteht ein Bedarf an hochaktiven Initiatoren, die schneller zerfallen als die derzeit vorwiegend eingesetzten Peroxydicarbonate und somit auch bei niedrigen Polymerisationstemperaturen kurze Reaktionszyklen erlauben. Zwar werden auch bisher schon höher aktive Initiatoren verwendet, wie ACSP (Acetylcyclohexansulfonylperoxid) oder CUPND (Cumylperoxyneodecanoat), doch weisen diese Initiatoren nachteilige Eigenschaften auf: unangenehmen Geruch der Zerfallsprodukte beim CUPND, bzw. korrosive Eigenschaften beim ACSP.

Darüber hinaus besteht bei flüssigen, hochaktiven Initiatoren die Notwendigkeit der Lagerung und des Transports bei tiefen Temperaturen, im allgemeinen unter -15 °C (EP 0 095 860).

Aus EP 271 462 (Kenobel AB) sind bei Umgebungstemperatur feste Ester der Monoperoxyoxalsäure bekannt. Es handelt sich um O-n-Alkyl,-OO-t-Alkyl-Ester der Monoperoxyoxalsäure, wobei die n-Alkylgruppen einen Rest mit 18 bis 28 C-Atomen darstellen.

Nachteilig an diesen Verbindungen ist, dass es sich um wachsartige Feststoffe handelt. Ihr Gehalt an Aktivsauerstoff ist durch das hohe Formelgewicht relativ gering (z.B. beim n-Docosyl-tert.butylperoxy-oxalat: 3,23%). Zu ihrer Herstellung muss man von langkettigen Fettalkoholen ausgehen, die in den erforderlichen unpolaren Lösemitteln nicht gut löslich sind. Man benötigt deshalb große Mengen an Lösemittel, das anschließend im Vakuum wieder abdestilliert werden muss.

Es sind zwar andere, bei Umgebungstemperatur feste Ester der Monoperoxyoxalsäure hergestellt worden, welche ein niedrigeres Formelgewicht und damit einen höheren Aktivsauerstoffgehalt aufweisen, jedoch ergaben sich dann Probleme mit den Sicherheitseigenschaften wie Schlagempfindlichkeit und Explosionsfähigkeit. Extrem ausgeprägt sind diese nachteiligen Eigenschaften beim Bis-tert.butylperoxy-oxalat.

Die Aufgabe der vorliegenden Erfindung bestand darin, einen bei Umgebungstemperatur festen, in Lösung hochaktiven Initiator bereitzustellen, der bei Temperaturen von etwa +10 °C sicher zu handhaben ist, ohne die Nachteile der bisher bekannten Initiatoren aufzuweisen.

Diese Aufgabe wurde erfindungsgemäß gelöst durch die Bereitstellung des neuen t-Butylperoxy-cyclododecyl-oxalats. Diese Verbindung ist eine bei 48 °C schmelzende kristalline Substanz mit einem Gehalt an Aktivsauerstoff von 4,8 % und einer Halbwertszeit von 10 Stunden bei 32 °C. Bei +18 °C beträgt der Verlust an Aktivsauerstoff ca. 3 % relativ in einer Woche, sodass die Verbindung bei Umgebungstemperatur für eine gewisse Zeitdauer sicher gehandhabt werden kann.

Überraschenderweise stellt t-Butylperoxy-cyclododecyl-oxalat einen hochaktiven Initiator für die Polymerisation von ungesättigten Monomeren und insbesondere für die Polymerisation von Vinylchlorid dar, welcher schneller zerfällt als die bisher bekannten Initiatoren und somit auch bei niedrigen Polymerisationstemperaturen von ≤ 50 °C kurze Reaktionszyklen erlaubt.

Ein weiterer Gegenstand der Anmeldung ist ein Verfahren zur Herstellung von t-Butylperoxy-cyclododecyl-oxalat, wobei man t-Butylhydroperoxid und Cyclododecyl-oxalyl-chlorid umsetzt und so das t-Butylperoxy-cyclodecyl-oxalat gewinnt. Bevorzugt wird das t-Butylperoxy-cyclododecyl-oxalat als kristalline Verbindung gewonnen, welche als feste Substanz bei Umgebungstemperaturen im wesentlichen stabil ist und somit ohne aufwendige Sicherheitsvorkehrungen gelagert und transportiert werden kann. Bei Zugabe des kristallinen t-Butylperoxy-cyclododecyl-oxalats zu einem geeigneten Lösungsmittel erhält man einen in Lösung hochaktiven Initiator, mit welchem die Polymerisation von ungesättigten Monomeren schneller als mit bisher verwendeten Initiatoren durchgeführt werden kann. Bei der Vinylchloridpolymerisation bei 40 bis 45 °C ist t-Butylperoxy-cyclododecyl-oxalat aktiver als die bekannten Initiatoren CUPND und ACSP.

Ein weiterer Gegenstand der Anmeldung ist die Verwendung von t-Butylperoxy-cyclododecyl-oxalat als Initiator für die Polymerisation von ungesättigten Monomeren, insbesondere von Vinylchlorid. t-Butylperoxy-cyclododecyl-oxalat kann aber auch als Initiator für andere ungesättigte Monomere eingesetzt werden und ist beispielsweise für die Herstellung von Polyvinylacetat und LDPE (low density polyethylene) geeignet.

Die Erfindung wird durch die folgenden Beispiele weiter erläutert.

### Beispiel 1:

### Herstellung von t-Butylperoxy-cyclododecyl-oxalat in wasserfreiem Medium

Zu einer Mischung aus 800 ml Methyl-t-butylether, 235 g (=1,2 Mol) einer wasserfreien 46,2 % - Lösung von t-Butylhydroperoxid in Petrolether (Sdp.40 °C) und 132 g (=1,2 Mol) 2,6-Dimethylpyridin werden bei -5 bis 0 °C 280 g (= 1,0 Mol) Cyclododecyl-oxalyl-chlorid (98,6 % Reinheit) unter Rühren und Kühlen zugetropft. Es bildet sich eine dickflüssige Suspension, die noch 45 Minuten bei 0 °C weitergerührt wird. Man gibt 600 ml Eiswasser zu, trennt die wässrige Phase nach 10 Minuten Stehen ab und wäscht die organische Phase zweimal mit je 400 ml kalter 5 % Schwefelsäure und zweimal mit je 400 ml Eiswasser.

Nach Abkühlen auf -25 °C kristallisiert der Perester aus. Man filtriert ab, wäscht mit wenig kaltem Petrolether und trocknet 2 Stunden bei 20 °C.

Man erhält 253 g eines weißen, feinkristallinen Pulvers mit einem Gehalt von 88 % an dem gewünschten Perester, das entspricht 68 % der theoretischen Ausbeute.

### Beispiel 2:

### Herstellung von t-Butylperoxy-cyclododecyl-oxalat in wässrigem Medium

Zu 800 ml Wasser gibt man 6 g eines kationischen Netzers (Dimethyl-fettalkyl-benzyl-ammoniumchlorid), 254 g (=2,2 Mol) 78 % wässriges t-Butylhydroperoxid und 242 g (=2,2 Mol) 2,6-Dimethylpyridin, kühlt die Mischung auf 0 °C und tropft unter Rühren und Kühlen innerhalb von 35 Minuten 489 g (=1,76 Mol) Cyclododecyl-oxalylchlorid (98.9 %) bei 0 bis +6 °C zu. Man rührt noch 45 Minuten bei +6 °C, gibt 600 ml Eiswasser und 90 ml 72 % Schwefelsäure zu, filtriert den Feststoff ab und wäscht ihn mit Eiswasser neutral und chloridfrei.

Man erhält 668 g eines weißen, feinkristallinen, wasserfeuchten Pulvers mit einem Gehalt an t-Butylperoxy-cyclododecyl-oxalat von 56,7 %, das entspricht 65,5 % der theoretischen Ausbeute.

### Beispiel 3:

### Vinylchlorid (VC) - Polymerisation mit t-Butylperoxy-cyclododecyl-oxalat im Vergleich zu ACSP

Das nach Beispiel 1 hergestellte t-Butylperoxy-cyclododecyl-oxalat (88 %) wurde auf seine Polymerisationswirksamkeit bei der Polymerisation von Vinylchlorid geprüft. Es wurde bei Temperaturen von 40 °C, 45 °C und 50 °C und bei Polymerisationszeiten von 2 Stunden, 4 Stunden und 6 Stunden in einem Stahlautoklaven (Suspensionspolymerisation) gearbeitet. Als Vergleich diente ACSP (Acetylcyclohexansulfonylperoxid). Die Ergebnisse sind in Tabelle 1 dargestellt, wobei CD-Peroxalat für t-Butylperoxy-cyclododecyl-oxalat steht.

| Initiator | T (°C) | Dosierung mMol 100 % Peroxid pro 70 g VC | Umsatz (%) nach | | |
|---|---|---|---|---|---|
| | | | 2 h | 4h | 6h |
| CD-Peroxalat | 40 | 0,12 | 12,1 | 33,2 | 50,8 |
| ACSP | 40 | 0,12 | 8,2 | 21,3 | 35,7 |
| CD-Peroxalat | 45 | 0,12 | 21,1 | 45,9 | 64,4 |
| ACSP | 45 | 0,12 | 13,3 | 32,1 | 47,8 |
| CD-Peroxalat | 50 | 0,12 | 29,3 | 48,6 | 56,7 |
| ACSP | 50 | 0,12 | 20,3 | 40,0 | 50,5 |

Wie aus Tabelle 1 ersichtlich, wird durch die Verwendung des erfindungsgemäßen t-Butylperoxy-cyclododecyl-oxalats bei den angegebenen Polymerisationstemperaturen ein deutlich erhöhter Umsatz im Vergleich zu ACSP erhalten.

## Patentansprüche

1. t-Butylperoxy-cyclododecyl-oxalat.

2. Verfahren zur Herstellung von t-Butylperoxy-cyclododecyl-oxalat,
**dadurch gekennzeichnet,**
**dass** man t-Butylhydroperoxid und Cyclododecyl-oxalylchlorid umsetzt und t-Butylperoxy-cyclododecyl-oxalat gewinnt.

3. Verfahren nach Anspruch 1,
**dadurch gekennzeichnet,**
**dass** man t-Butylperoxy-cyclododecyl-oxalat als kristalline Verbindung gewinnt.

4. Verwendung von t-Butylperoxy-cyclododecyl-oxalat als Initiator für die Polymerisation von ungesättigten Monomeren.

5. Verwendung nach Anspruch 4,
**dadurch gekennzeichnet,**
**dass** man als Monomere Vinylchlorid verwendet.

## Claims

1. t-Butylperoxy-cyclododecyl oxalate.

2. Process for producing t-butylperoxy-cyclododecyl oxalate,
**characterized in that**
t-butylhydroperoxide and cyclododecyl oxalyl chloride are reacted to obtain t-butylperoxy-cyclododecyl oxalate.

3. Process as claimed in claim 1,
**characterized in that**
t-butylperoxy-cyclododecyl oxalate is obtained as a crystalline compound.

4. Use of t-butylperoxy-cyclododecyl oxalate as an initiator for the polymerization of unsaturated monomers.

5. Use as claimed in claim 4,
**characterized in that**
vinyl chloride is used as the monomer.

## Revendications

1. t-butyleperoxy-cyclododécyl-oxalate.

2. Procédé pour fabriquer du t-butyleperoxy-cyclododécyl-oxalate **caractérisé en ce que** l'on transforme du t-butyle-hydroperoxyde et du chlorure de cyclododécyl-oxalyle et qu'on obtient le t-butyleperoxy-cyclododécyl-oxalate.

3. Procédé selon la revendication 1
**caractérisé en ce que** l'on obtient le t-butyleperoxy-cyclododécyl-oxalate en tant que composé cristallin.

4. Utilisation de t-butyleperoxy-cyclododécyl-oxalate en tant qu'initiateur pour polymériser des monomères insaturés.

5. Utilisation selon la revendication 4
**caractérisée en ce que** l'on utilise du chlorure de vinyle comme monomère.
